# FASCICULE DE BREVET EUROPEEN

(11) **EP 0 756 472 B1**
(45) Date de publication et mention de la délivrance du brevet: **28.07.1999**
(21) Numéro de dépôt: 95918025.8
(22) Date de dépôt: 19.04.1995
(51) Int. Cl.: A61B 17/70

(54) **DISPOSITIF DE STABILISATION D'ANCRAGE ORTHOPEDIQUE**
STABILISIERUNGSVORRICHTUNG FÜR ORTHOPÄDISCHE VERANKERUNGSVORRICHTUNG
DEVICE FOR STABILIZING ORTHOPEDIC ANCHORS

(30) Priorité: 20.04.1994 FR 9405039
(43) Date de publication de la demande: 05.02.1997
(73) Titulaire: ROUSSOULY, Pierre, 69450 Saint Cyr au Mont d'Or (FR); TAGLANG, Gilbert, 67370 Griesheim sur Souffel (FR); GROSSE, Arsène, 67400 Illkirch-Graffenstaden (FR); CHOPIN, Daniel, 62155 Merlimont (FR)
(72) Inventeur: ROUSSOULY, Pierre, 69450 Saint Cyr au Mont d'Or (FR); TAGLANG, Gilbert, 67370 Griesheim sur Souffel (FR); GROSSE, Arsène, 67400 Illkirch-Graffenstaden (FR); CHOPIN, Daniel, 62155 Merlimont (FR)
(74) Mandataire: Moncheny, Michel
(86) Numéro de dépôt international: FR9500507
(87) Numéro de publication internationale: WO9528889

(56) Documents cités:
- WO-A-93/20771
- WO-A-94/00062
- FR-A- 2 275 679
- FR-A- 2 642 642
- GB-A- 2 173 104
- US-A- 4 289 123
- US-A- 5 269 784

## Description

La présente invention se rapporte aux ancrages tels que les vis pédiculaires et les crochets utilisés pour l'instrumentation du rachis, qui sont placés sur les vertèbres pour constituer des éléments d'ancrage reliés les uns aux autres par une ou plusieurs tiges de solidarisation.

Les crochets orthopédiques que l'on utilise habituellement pour l'instrumentation du rachis sont conformés pour s'accrocher sur une partie de vertèbre. Un tel crochet comprend un corps de crochet comportant une face intérieure d'appui et une face extérieure de fixation munie de moyens de fixation à une tige de solidarisation de points d'ancrage, et une lame incurvée formant avec la face intérieure de corps un profil en U pour envelopper ladite partie de vertèbre. La lame incurvée comprend une branche de traction généralement opposée à la face intérieure de corps à laquelle elle se raccorde par une branche de liaison.

Ces crochets orthopédiques utilisés pour l'instrumentation du rachis sont placés sur les vertèbres de trois façons différentes.

Selon un premier mode d'utilisation, ils sont orientés vers le haut : ils sont glissés entre deux articulaires et prennent appui par l'extrémité échancrée de leur lame sur le pédicule de la vertèbre, et par le creux de leur profil en U sous l'épaulement de l'articulaire inférieur et de la lame en dedans.

Selon un second mode d'utilisation, ils sont orientés vers le haut : ils sont glissés sous la lame vertébrale et prennent appui par le creux de leur profil en U sur le bord inférieur de la lame de la vertèbre.

Selon un troisième mode d'utilisation, ils sont orientés vers le bas, et ils prennent alors appui par le creux de leur profil en U sur le bord supérieur de la lame de la vertèbre.

La problème est que, quelle que soit leur position, les crochets n'ont pas ou très peu de stabilité primaire, c'est-à-dire qu'ils ne constituent un élément d'ancrage stable qu'à condition d'être combinés avec un second élément d'ancrage tel qu'un autre crochet en opposition ou une vis de fixation. Même en présence d'un autre élément d'ancrage en opposition, un tel crochet peut glisser médialement sur la lame et échapper de l'appui du pédicule. Il peut tourner en dedans et passer dans le canal médullaire de la vertèbre, devenant dangereux pour la moelle épinière.

Dans certaines vertèbres, on réalise un ancrage au moyen d'une vis à os vissée dans le pédicule de la vertèbre. Une telle vis pédiculaire comprend une partie externe filetée dont le filetage à pas faible est adapté pour le vissage d'un écrou, un corps d'ancrage ou partie intermédiaire de plus grand diamètre, et une partie interne filetée coaxiale dont le filetage à pas réduit est adapté pour se visser dans le pédicule et dans le corps vertébral. La vis est reliée à une tige de liaison par une pince serrée par l'écrou vissé sur la partie externe filetée. En alternative, la vis est reliée à une plaque ou bande de liaison dont une portion est traversée par la partie externe filetée de vis et serrée par l'écrou vissé sur ladite partie externe filetée.

Lors de la pose, on visse la partie interne filetée dans l'os, puis on adapte la tige ou plaque de liaison sur la partie externe filetée, et on visse l'écrou pour réaliser la réduction de déformation vertébrale et la solidarisation de la tige ou plaque de liaison et de la vis. Le serrage de l'écrou induit un couple de rotation sur la vis pédiculaire, ce qui produit parfois un serrage excessif de la vis dans l'os.

Pendant la réduction et pendant l'utilisation ultérieure, les mouvements et contraintes répétés du rachis provoquent parfois un léger dévissage de la partie interne filetée, ou un retrait axial de la vis, ou encore un déchaussement résultant des oscillations et contraintes latérales répétées qui font osciller la vis lorsque seule la partie intermédiaire de sa partie interne filetée est en prise sur l'os cortical du pédicule. L'ancrage est alors fragilisé.

Les documents GB-A-2 173 104 et FR-A-2 275 679 décrivent des éléments d'ancrage orthopédique dans lesquels une rondelle à dents courtes est insérée entre le corps d'ancrage et l'os de vertèbre, les dents s'engageant dans l'os. Une telle rondelle n'interdit pas les rotations relatives, et n'assure donc pas une stabilisation primaire de l'ancrage.

Le document US-A-5 269 784 décrit un ancrage comprenant un écrou écarteur. Un tel écrou ne peut pas, non plus, assurer un blocage en rotation pour stabilisation primaire de l'ancrage.

Le document FR-A-2 642 642 décrit un crochet d'ancrage orthopédique associé à une vis pédiculaire assurant son maintien sur une vertèbre.

Le document WO-A-94 00062 décrit un dispositif de stabilisation d'ancrage orthopédique selon le préambule de la revendication 1, avec :
- un corps d'ancrage comportant une face intérieure d'appui pour porter contre la vertèbre et une face extérieure opposée de fixation,
- des moyens d'ancrage, solidaires du corps d'ancrage, et conformés pour se fixer à la vertèbre,
- une partie de fixation, solidaire du corps d'ancrage, et munie de moyens de fixation à une tige ou plaque de solidarisation de points d'ancrage.

### EXPOSE DE L'INVENTION

Le problème proposé par la présente invention est d'assurer la stabilisation primaire de l'ancrage orthopédique sur la vertèbre, pour améliorer l'ancrage osseux dès l'étape préliminaire de pose de l'instrumentation sur le rachis, pendant l'étape de réduction de déformation du rachis, et pendant toute la durée ultérieure de maintien du rachis. On cherche à obtenir de l'ancrage, qu'il soit sous forme de vis pédiculaire ou de crochet, une stabilité fiable et permanente, pour éviter les pertes de réduction à court ou moyen terme. Il convient pour cela d'interdire toute rotation ou toute oscillation de la vis d'ancrage, toute rotation ou toute translation du crochet d'ancrage.

La stabilisation d'un crochet sur une vertèbre est a priori délicate. En effet, si l'on tente de stabiliser le crochet à l'aide d'une vis osseuse vissée dans la vertèbre, encore faut-il trouver une partie de vertèbre solide pour recevoir la vis : cela est rarement le cas ; en particulier, l'os de la lame vertébrale, qui est naturellement mince et fragile, n'est pas adapté pour recevoir une vis osseuse. Une solution alternative consiste à prévoir deux crochets en opposition l'un de l'autre et reliés l'un à l'autre par un dispositif de serrage permettant de les rapprocher l'un de l'autre parallèlement à leur face intérieure d'appui. Une telle solution s'avère peu commode d'emploi, car il faut trouver un appui correct des deux crochets simultanément, et la stabilisation latérale est insuffisante.

L'invention vise à réaliser une stabilisation améliorée, empêchant la rotation primaire du crochet sous l'apophyse articulaire et le pédicule de vertèbre, dans le cas d'un crochet pédiculaire. L'invention vise également à empêcher la rotation primaire et la translation primaire du crochet sur la lame vertébrale. Cette stabilisation est assurée pendant et après la réduction de déformation du rachis.

L'invention vise également à assurer la stabilisation primaire d'une vis pédiculaire, en évitant les oscillations latérales de la vis, et empêchant son dévissage hors de l'os et son vissage excessif dans l'os, et empêchant son retrait hors de l'os.

Pour atteindre ces objets ainsi que d'autres, le dispositif de stabilisation d'ancrage orthopédique selon l'invention s'adapte sur un ancrage en forme de crochet ou de vis. L'ancrage comprend :
- un corps d'ancrage comportant une face intérieure d'appui pour porter contre la vertèbre et une face extérieure opposée de fixation,
- des moyens d' ancrage, solidaires du corps d' ancrage, et conformés pour se fixer à la vertèbre,
- une partie de fixation, solidaire du corps d'ancrage, et munie de moyens de fixation à une tige ou plaque de solidarisation de points d'ancrage.

Le dispositif de stabilisation comprend une agrafe de stabilisation comportant :
- un corps d'agrafe , adapté pour s'engager sur la face extérieure de fixation du corps d'ancrage, et muni de moyens de solidarisation au corps d'ancrage, s'opposant aux rotations relatives entre le corps d'agrafe et le corps d' ancrage, et permettant sélectivement de rapprocher le corps d'agrafe du corps d'ancrage jusqu'à une position de stabilisation en appui contre la face extérieure de fixation et d'éloigner le corps d'agrafe du corps d'ancrage jusqu'à une position de libération,
   - au moins une dent d'agrafe, solidaire du corps d'agrafe, orientée et conformée pour pénétrer dans la vertèbre au delà de la face intérieure d'appui du corps d'ancrage lorsque l'agrafe est en position de stabilisation sur le corps de l'ancrage lui-même adapté en position d'ancrage sur la vertèbre.

Selon un premier mode de réalisation, le corps d'agrafe est articulé sur le corps d'ancrage selon un axe transversal.

Selon un second mode de réalisation, l'agrafe est une pièce amovible distincte de l'ancrage, adaptée pour s'engager sur la face extérieure de fixation du corps d'ancrage.

Dans tous les cas, on peut avantageusement prévoir une partie de fixation d'ancrage interdisant toute rotation axiale relative entre le corps d'ancrage et la tige de solidarisation de points d'ancrage. Pour cela, selon un mode de réalisation avantageux :
- la partie de fixation d'ancrage est une tige filetée dépassant de la face extérieure de fixation du corps d'ancrage, et destinée à recevoir un cavalier de serrage et un écrou,
- des premiers reliefs antiglissement sont ménagés sur la face extérieure de fixation du corps d'ancrage,
- des reliefs antiglissement intermédiaires correspondants sont ménagés sur les deux faces opposées du corps d'agrafe.
- des seconds reliefs antiglissement sont ménagés sur une face du cavalier de serrage.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles:
- la figure 1 est une première vue éclatée en perspective d'un élément d'ancrage comportant un crochet orthopédique et un dispositif de stabilisation selon un premier mode de réalisation de l' invention ;
- la figure 2 est une seconde vue éclatée en perspective du dispositif de la figure 1 ;
- la figure 3 est une vue en perspective du dispositif de la figure 1, assemblé en position d' ancrage ;
- la figure 4 est une vue en perspective montrant un dispositif de stabilisation de crochet orthopédique selon un second mode de réalisation de l'invention ;
- la figure 5 est une vue en perspective illustrant un élément d'ancrage avec dispositif de stabilisation selon l'invention, dans un autre mode de réalisation ;
- la figure 6 illustre en perspective une structure de dispositif de stabilisation de crochet orthopédique selon un autre mode de réalisation de l'invention ;
- la figure 7 est une vue en perspective d'un autre mode de réalisation de dispositif de stabilisation de crochet orthopédique selon l' invention ; et
- la figure 8 est une vue en perspective illustrant une agrafe de stabilisation selon un mode de réalisation adapté à la stabilisation d'une vis pédiculaire.

### DESCRIPTION DES MODES DE REALISATION PREFERES

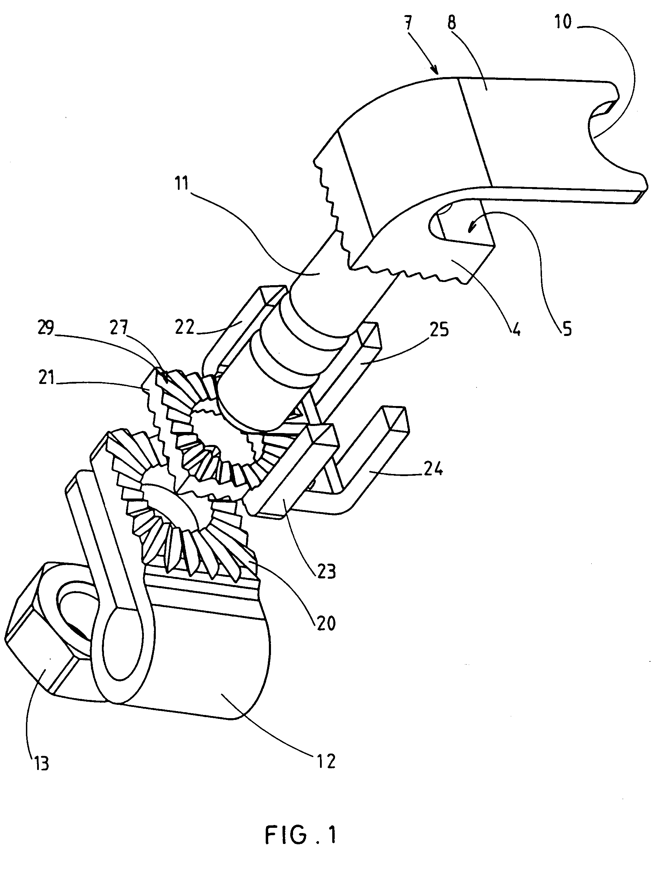

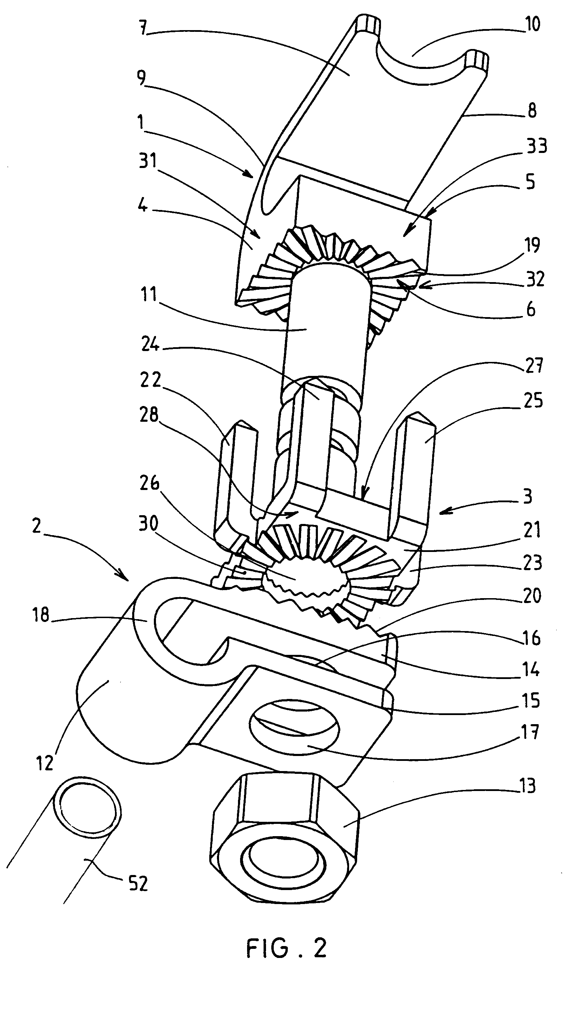

Dans le mode de réalisation illustré sur les figures 1 à 3, un élément d'ancrage à dispositif de stabilisation de crochet selon l'invention comprend un crochet orthopédique 1, des moyens de fixation 2 du crochet sur une tige de solidarisation 52 de points d'ancrage, et un dispositif de stabilisation 3 pour stabiliser le crochet 1 sur une vertèbre.

Le crochet 1 comprend un corps de crochet 4 comportant une face intérieure d'appui 5 et une face extérieure de fixation 6 munie des moyens de fixation 2 à la tige de solidarisation 52. Le crochet 1 comprend en outre une lame incurvée 7, formant avec la face intérieure 5 de corps de crochet 4 un profil en U destiné à envelopper une partie de vertèbre sur laquelle le crochet 1 doit s'ancrer. La lame incurvée 7 comprend une branche de traction 8 généralement opposée et parallèle à la face intérieure d'appui 5 du corps de crochet 4, à laquelle elle se raccorde par une branche de liaison 9. L'extrémité libre de la branche de traction 8 comprend avantageusement une échancrure 10, destinée à favoriser le positionnement du crochet sous l'apophyse articulaire et le pédicule d'une vertèbre.

Dans le mode de réalisation représenté sur les figures, les moyens de fixation 2 comprennent une tige de fixation 11 filetée, dépassant de la face extérieure 6 de fixation du corps de crochet 4, et destinée à recevoir un cavalier de serrage 12 et un écrou 13. Le cavalier de serrage 12 comprend deux branches 14 et 15 munies de trous respectifs 16 et 17 de passage de la tige de fixation 11, les branches 14 et 15 parallèles se raccordant l'une à l'autre par une partie intermédiaire incurvée 18 destinée à entourer une partie de la tige de solidarisation 52. Par serrage de l'écrou 13, on rapproche les branches 14 et 15 du cavalier de serrage 12, qui se serre autour de la tige de solidarisation 52, pour solidariser le crochet 1 sur ladite tige de solidarisation 52.

Pour s'opposer aux rotations du crochet 1 par rapport à la tige de solidarisation 52, on prévoit des premiers reliefs antiglissement 19 sur la face extérieure 6 de fixation du corps de crochet 4, et des seconds reliefs antiglissement 20 sur la face correspondante de la branche 14 du cavalier de serrage 12.

Le dispositif de stabilisation 3 comprend, dans ce mode de réalisation représenté sur les figures 1 à 3, une agrafe de stabilisation comportant un corps d'agrafe 21 muni de moyens de solidarisation au corps de crochet 4, et quatre dents d'agrafe 22, 23, 24 et 25 solidaires du corps d'agrafe 21 et se développant perpendiculairement au corps d'agrafe 21 dans la même direction. La ou les dents d'agrafe 22-25, solidaires du corps d'agrafe 21, se développent dans l'intérieur du profil en U en direction de la branche de traction 8 sensiblement perpendiculairement à la face intérieure d'appui 5 du corps de crochet 4 pour pénétrer, en position de maintien, dans ladite partie de vertèbre.

Dans le mode de réalisation représenté, en position de maintien de l'agrafe 3 sur le crochet 1, l'extrémité libre des dents 22-25 est légèrement en retrait de la branche de traction 8 du crochet 1.

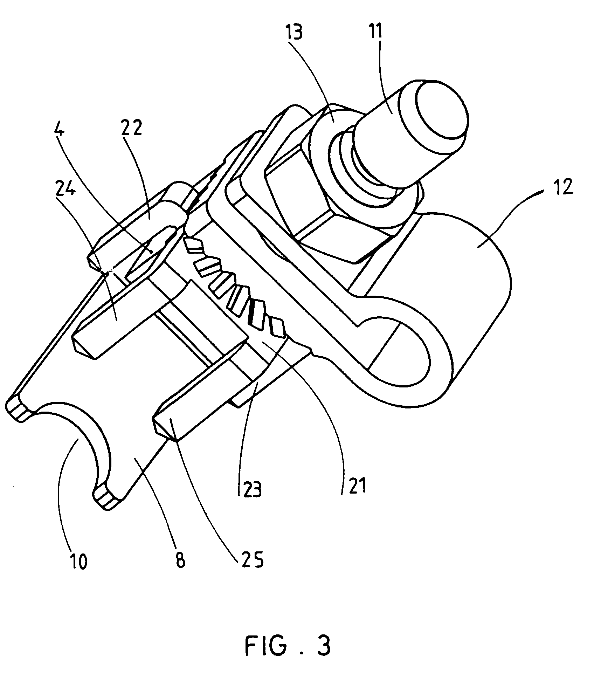

Les moyens de solidarisation du corps d'agrafe 21 sur le corps de crochet 4 utilisent la tige de fixation 11, qui traverse un trou 26 ménagé dans le corps d'agrafe 21, la tige de fixation 11 étant alors sensiblement parallèle aux quatre dents 22-25 de l'agrafe. Lors de l'utilisation, on dispose le corps d'agrafe 21 autour de la tige de fixation 11 et sur la face extérieure de fixation 6, en orientant les dents 22-25 en direction du crochet 1. Les moyens de solidarisation du corps d'agrafe 21 au corps de crochet 4 permettent ainsi un déplacement relatif du corps d'agrafe 21 vers et à l'écart du corps de crochet 4, entre une position de retrait dans laquelle les dents 22-25 sont à l'écart de la lame incurvée 7 de crochet et une position de stabilisation illustrée sur la figure 3 dans laquelle les dents 22-25 se développent dans l'intérieur du profil en U de crochet 1 en direction de la branche de traction 8, sensiblement perpendiculairement à la face intérieure d'appui 5 du corps de crochet 4, pour pénétrer dans la partie de vertèbre insérée dans le creux du crochet 1. En position de stabilisation, le corps d'agrafe 21 vient en appui par sa première face 27 sur la face extérieure de fixation 6 du corps de crochet 4, et le corps d'agrafe 21 est repoussé vers le crochet 1 par le cavalier de serrage 12 dont la face d'appui à reliefs antiglissement 20 vient en appui contre la seconde face 28 du corps d'agrafe 21. Pour s'opposer aux rotations relatives entre le crochet 1 et le cavalier de serrage 12 solidaire de la tige de solidarisation, on réalise des troisièmes reliefs antiglissement 29 sur la première face 27 du corps d'agrafe 21, et des quatrièmes reliefs antiglissement 30 sur la seconde face 28 du corps d'agrafe 21.

Les dents 22-25 sont positionnées de façon que le dispositif de stabilisation vienne chevaucher le corps de crochet 4. Dans le mode de réalisation représenté, les dents 22 et 23 sont deux dents latérales disposées pour dépasser au-delà des bords latéraux 31 et 32 du corps de crochet 4. Les dents 24 et 25 sont deux dents frontales dépassant au-delà du bord d'extrémité libre 33 du corps de crochet 4.

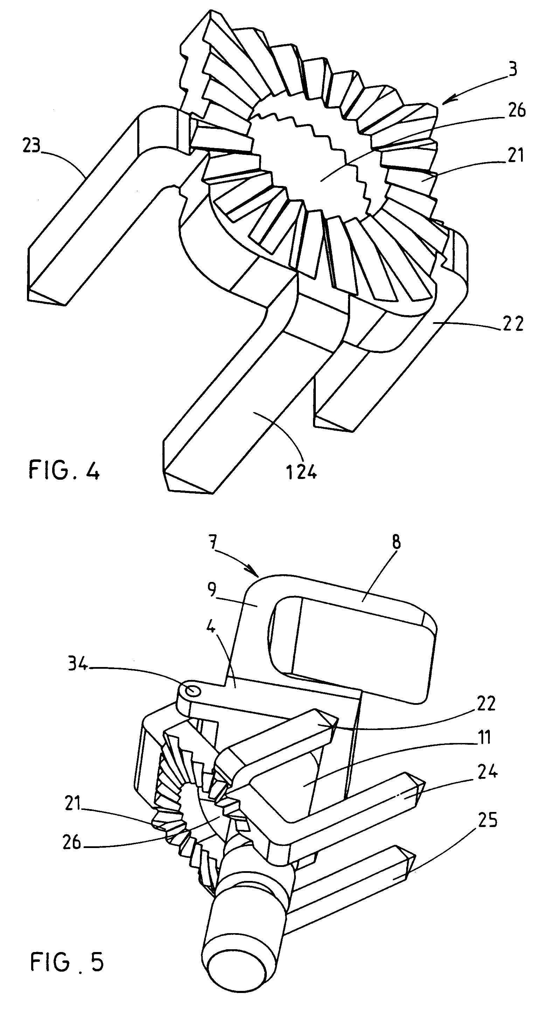

La figure 4 illustre un mode de réalisation légèrement différent du dispositif de stabilisation 3, dans lequel on retrouve un corps d'agrafe 21 similaire du corps d'agrafe des figures 1 à 3, et deux dents latérales d'agrafe 22 et 23 similaires des dents latérales du mode de réalisation des figures 1 à 3. La différence est que ce dispositif de stabilisation 3 de la figure 4 comprend une seule dent frontale 124, placée en position sensiblement centrale et comme illustré sur la figure.

Dans les modes de réalisation des figures 1 à 4, l'agrafe de stabilisation constitue une pièce amovible distincte du crochet 1, adaptée pour s'engager sur la face extérieure de fixation 6 du corps de crochet 4.

La figure 5 illustre un mode de réalisation différent selon l'invention, dans lequel le corps d'agrafe 21 est articulé sur le corps de crochet 4 selon un axe transversal 34 disposé au voisinage de la partie de raccordement du corps de crochet 4 à la lame incurvée 7. Le trou 26 de passage de la tige de fixation 11 dans le corps d'agrafe 21 est ouvert entre les dents frontales 24 et 25, pour permettre le basculement de l'agrafe comme illustré sur la figure.

Pour le reste, le dispositif d'ancrage de la figure 5 comprend les mêmes éléments que le dispositif d'ancrage des figures 1 à 3, et ces éléments sont repérés par les mêmes références numériques.

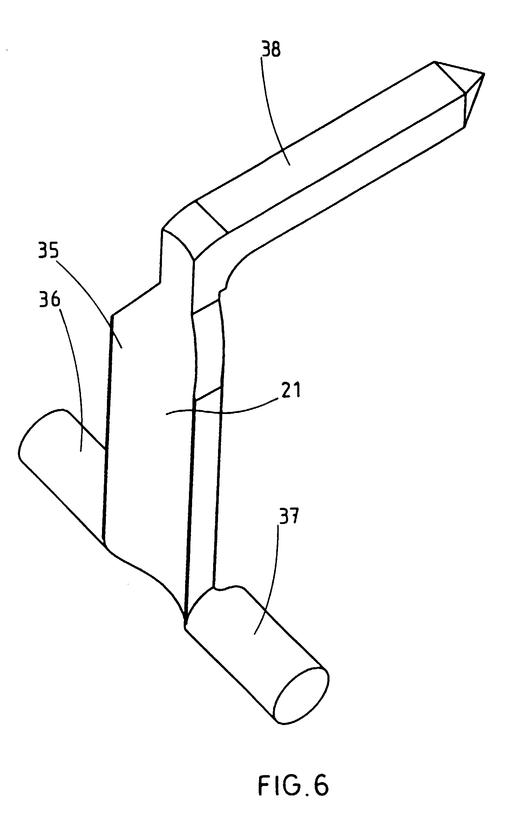

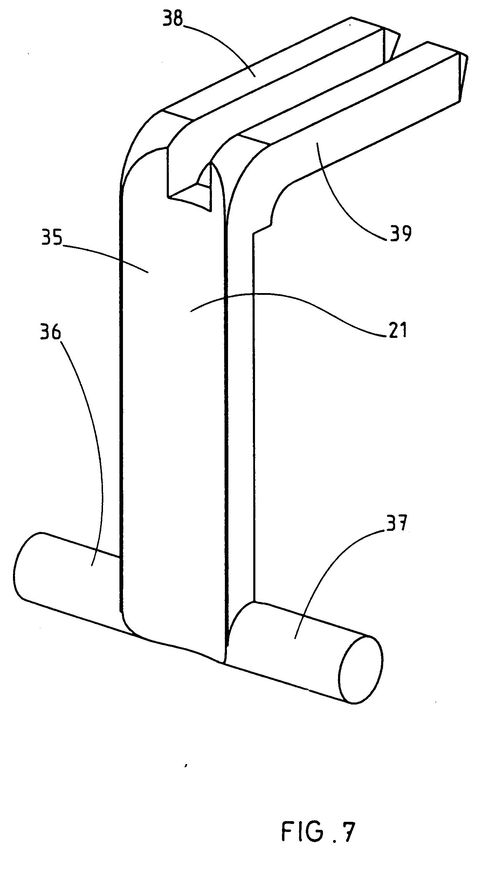

Les figures 6 et 7 illustrent deux autres modes de réalisation selon l'invention pour le dispositif de stabilisation 3 d'un crochet 1 sur les vertèbres. Sur la figure 6, le corps d'agrafe 21 présente une forme en T, avec une branche centrale 35 et deux branches latérales 36 et 37 à section circulaire. Le corps d'agrafe se raccorde à une dent unique 38 frontale. Une telle forme d'agrafe est destinée à s'adapter sur des crochets dont les moyens de fixation à une tige de solidarisation comprennent un corps de crochet muni d'une fente diamétrale de passage de la tige de solidarisation, avec des moyens de serrage de la tige dans le corps de crochet. La forme du corps d'agrafe 21 permet son insertion dans la fente diamétrale de passage de tige de solidarisation.

Le mode de réalisation de la figure 7 reprend la même forme de corps d'agrafe 21, avec une branche centrale 35 et deux branches latérales 36 et 37. La différence est que l'on prévoit deux dents frontales 38 et 39 parallèles l'une à l'autre, comme illustré sur la figure.

Dans tous les modes de réalisation qui ont été précédemment décrits, le corps d'agrafe 21 est adapté pour que les moyens de fixation du crochet 1 à une tige de solidarisation assurent également la solidarisation de l'agrafe 3 sur le crochet 1, en repoussant les dents 22-25 en direction de la lame incurvée 7 pour pénétrer dans une partie de vertèbre insérée dans le creux du crochet 1. On assure ainsi une stabilisation primaire efficace du crochet 1 sur une vertèbre.

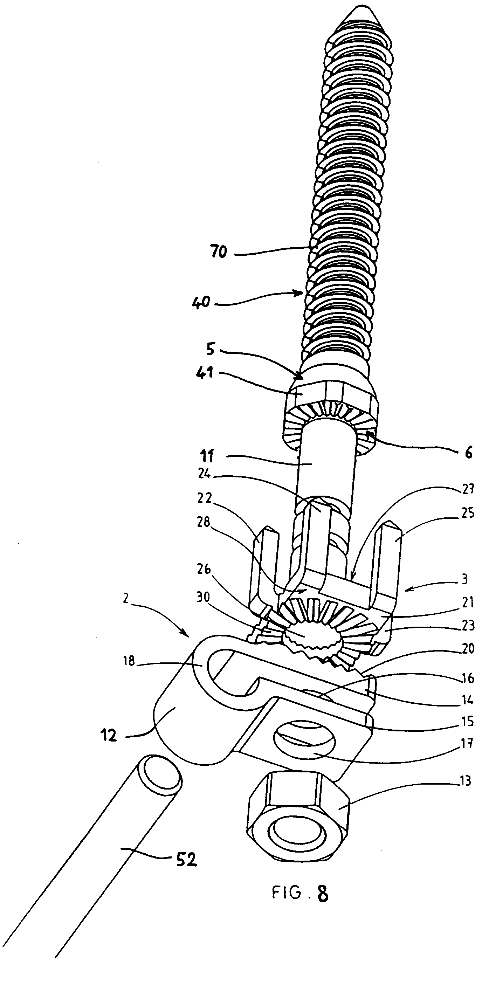

Dans le mode de réalisation illustré sur la figure 8, le dispositif de stabilisation selon l'invention est adapté pour stabiliser un ancrage réalisé par une vis pédiculaire 40. La vis pédiculaire 40 comprend un corps d'ancrage 41 à face intérieure d'appui 5 et à face extérieure de fixation 6, avec une tige interne filetée 70 adaptée pour se visser dans l'os de vertèbre, et avec des moyens de fixation comportant une tige externe filetée 11 coaxiale adaptée pour le vissage d'un écrou 13 pour le serrage d'un cavalier de serrage 12 sur une tige de solidarisation 52 de points d'ancrage.

Pour la stabilisation d'un tel ancrage à vis pédiculaire, on utilise une agrafe de stabilisation 3, par exemple à quatre dents 22-25 telle qu'illustrée sur la figure 8 selon la même structure que dans le mode de réalisation des figures 1 à 3. La ou les dents d'agrafe 22-25, solidaires du corps d'agrafe 21, se développent axialement autour du corps d'ancrage 41 et au delà de la face intérieure d'appui 5 pour pénétrer dans l'os vertébral lorsque l'ancrage est en position sur la vertèbre avec l'agrafe 3 en appui sur la face extérieure de fixation 6.

Les agrafes de stabilisation illustrées sur les figures permettent de réaliser un élément d'ancrage orthopédique pour instrumentation du rachis, comprenant un ancrage orthopédique 1, tel qu'un crochet ou une vis pédiculaire, et une agrafe de stabilisation 3.

Selon l'invention, on réalise ainsi un appareil de traitement du rachis, comprenant au moins une tige ou plaque de solidarisation 52 reliant des éléments d'ancrage adaptés pour se fixer aux vertèbres du rachis, l'un au moins des éléments d'ancrage comprenant un ancrage orthopédique 1 tel qu'un crochet ou une vis pédiculaire, et une agrafe de stabilisation 3.

La présente invention n'est pas limitée aux modes de réalisation qui ont été explicitement décrits, mais elle en inclut les diverses variantes et généralisations contenues dans le domaine des revendications ci-après.

## Revendications

1. Dispositif de stabilisation d'ancrage orthopédique sur une vertèbre, pour instrumentation du rachis, l'ancrage comprenant :
- un corps d'ancrage (4, 41) comportant une face intérieure d'appui (5) pour porter contre la vertèbre et une face extérieure opposée de fixation (6),
- des moyens d'ancrage (7, 70), solidaires du corps d'ancrage (4,41), et conformés pour se fixer à la vertèbre,
- une partie de fixation (11), solidaire du corps d'ancrage (4,41), et munie de moyens de fixation à une tige ou plaque de solidarisation (52) de points d'ancrage,
caractérisé en ce qu'il comprend une agrafe de stabilisation (3) comportant :
- un corps d'agrafe (21), adapté pour s'engager sur la face extérieure de fixation (6) du corps d'ancrage (4,41), et muni de moyens de solidarisation (26,34 ; 36,37) au corps d'ancrage (4,41), s'opposant aux rotations relatives entre le corps d'agrafe (21) et le corps d'ancrage (4,41), et permettant sélectivement de rapprocher le corps d'agrafe (21) du corps d'ancrage (4,41) jusqu'à une position de stabilisation en appui contre la face extérieure de fixation (6) et d'éloigner le corps d'agrafe (21) du corps d'ancrage (4,41) jusqu'à une position de libération,
- au moins une dent d'agrafe (22-25 ; 38 ; 39 ; 124), solidaire du corps d'agrafe (21), orientée et conformée pour pénétrer dans la vertèbre au delà de la face intérieure d'appui (5) du corps d'ancrage (4,41) lorsque l'agrafe (3) est en position de stabilisation sur le corps de l'ancrage (4,41) lui-même adapté en position d'ancrage sur la vertèbre.

2. Dispositif selon la revendication 1, caractérisé en ce que le corps d'agrafe (21) est articulé sur le corps d'ancrage (4,41) selon un axe transversal (34).

3. Dispositif selon la revendication 1, caractérisé en ce que l'agrafe de stabilisation (3) est une pièce amovible distincte de l'ancrage, adaptée pour s'engager sur la face extérieure de fixation (6) du corps d'ancrage (4,41).

4. Dispositif selon l'une quelconque des revendications 1 à 3, caractérisé en ce que :
- la partie de fixation d'ancrage est une tige filetée (11) dépassant de la face extérieure (6) de fixation du corps d'ancrage (4,41), et destinée à recevoir un cavalier de serrage (12) et un écrou (13),
- des premiers reliefs antiglissement (19) sont ménagés sur la face extérieure de fixation (6) du corps d'ancrage (4,41),
- des reliefs antiglissement (29, 30) intermédiaires correspondants sont ménagés sur les deux faces opposées (27,28) du corps d'agrafe (21),
- des seconds reliefs antiglissement (20) sont ménagés sur une face du cavalier de serrage (12).

5. Dispositif de stabilisation d'ancrage orthopédique (1) selon l'une quelconque des revendications 1 à 4, dans lequel :
- l'ancrage est un crochet (1) comprenant un corps de crochet (4) comportant une face intérieure d'appui (5) et une face extérieure de fixation (6) munie de moyens de fixation (11, 12, 13) à une tige de solidarisation (52) de points d'ancrage, et une lame incurvée (7) formant avec la face intérieure (5) du corps (4) de crochet un profil en U pour envelopper une partie de vertèbre, avec une branche de traction (8) généralement opposée et parallèle à la face intérieure d'appui (5) de corps de crochet (4) à laquelle elle se raccorde par une branche de liaison (9),
- la ou les dents d'agrafe (22-25), solidaires du corps d'agrafe (21), se développent dans l'intérieur du profil en U en direction de la branche de traction (8) sensiblement perpendiculairement à la face intérieure d'appui (5) du corps de crochet (4) pour pénétrer, en position de maintien, dans ladite partie de vertèbre.

6. Dispositif selon la revendication 5, caractérisé en ce que l'agrafe de stabilisation (3) comprend deux dents latérales (22, 23) dépassant au-delà des bords latéraux (31, 32) du corps de crochet (4), et au moins une dent frontale (24, 25) dépassant au-delà du bord d'extrémité libre (33) du corps de crochet (4).

7. Dispositif selon la revendication 5, caractérisé en ce que l'agrafe de stabilisation (3) comprend deux dents latérales (22, 23) dépassant au-delà des bords latéraux (31, 32) du corps de crochet (4), et deux dents frontales (24, 25) dépassant au-delà du bord d'extrémité libre (33) du corps de crochet (4).

8. Dispositif selon l'une quelconque des revendications 5 à 7, caractérisé en ce que les dents (22, 25) ont une longueur telle que, en position de maintien de l'agrafe (3) sur le crochet (1), l'extrémité libre des dents est légèrement en retrait de la branche de traction (8) du crochet (1).

9. Dispositif de stabilisation d'ancrage orthopédique selon l'une quelconque des revendications 1 à 4, dans lequel :
- l'ancrage est une vis pédiculaire (40) comprenant un corps d'ancrage (41) à face intérieure d'appui (5) et à face extérieure de fixation (6), avec une tige interne filetée (70) adaptée pour se visser dans l'os de vertèbre, et avec des moyens de fixation (11, 12, 13) à une tige de solidarisation (52) de points d'ancrage,
- la ou les dents d'agrafe (22-25), solidaires du corps d'agrafe (21), se développent axialement autour du corps d'ancrage (41) et au delà de la face intérieure d'appui (5) pour pénétrer dans l'os vertébral lorsque l'ancrage est en position sur la vertèbre avec l'agrafe (3) en appui sur la face extérieure de fixation (6).

10. Appareil de traitement du rachis, comprenant au moins une tige ou plaque de solidarisation (52) reliant des éléments d'ancrage adaptés pour se fixer aux vertèbres du rachis, caractérisé en ce que l'un au moins des éléments d'ancrage comprend un ancrage orthopédique (1) et un dispositif de stabilisation d'ancrage orthopédique (3) selon l'une quelconque des revendications 1 à 9.

## Claims

1. Device for stabilising orthopaedic anchors on a vertebra, for instrumentation of the spine, the anchor comprising:
an anchor member (4, 41) comprising an inner bearing surface (5) for bearing on the vertebra and an opposite, outer fixing surface (6),
anchor means (7, 70) fixedly connected to the anchor member (4, 41) and shaped to be fixed to the vertebra,
a fixing member (11) fixedly connected to the anchor member (4, 41) and provided with means for fixing to an attachment pin or plate (52) for anchor points, characterised in that it comprises a stabilising clip (3) having:
a clip member (21) adapted to engage on the outer fixing surface (6) of the anchor member (4, 41), and provided with means of attachment (26, 34; 36, 37) to the anchor member (4, 41), countering the relative rotations between the clip member (21) and the anchor member (4, 41), and enabling the clip member (21) to be brought selectively closer to the anchor member (4, 41) into a stabilising position bearing on the outer fixing surface (6) and enabling the clip member (21) to be moved away from the anchor member (4, 41) into a release position,
at least one clip tooth (22-25; 38; 39; 124) fixedly attached to the clip member (21), oriented and shaped so as to engage in the vertebra beyond the inner abutment surface (5) of the anchor member (4, 41) when the clip (3) is in the stabilising position on the anchor member (4, 41) which is itself adjusted into the anchoring position on the vertebra.

2. Device according to claim 1, characterised in that the clip member (21) is articulated on the anchor member (4, 41) along a transverse axis (34).

3. Device according to claim 1, characterised in that the stabilising clip (3) is a movable part separated from the anchor, adapted to engage on the outer fixing surface (6) of the anchor member (4, 41).

4. Device according to any one of claims 1 to 3, characterised in that:
the anchor fixing portion is a threaded rod (11) projecting from the outer fixing surface (6) of the anchor member (4, 41) and adapted to receive a clamping staple (12) and a nut (13),
first non-slip embossed markings (19) are provided on the outer fixing surface (6) of the anchor member (4, 41),
corresponding intermediate non-slip embossed markings (29, 30) are provided on the two opposing surfaces (27, 28) of the clip member (21),
second non-slip embossed markings (20) are provided on one side of the clamping staple (12).

5. Device for stabilising orthopaedic anchors (1) according to any one of claims 1 to 4, wherein:
the anchor is a hook (1) comprising a hook member (4) having an inner bearing surface (5) and an outer fixing surface (6) provided with means of attachment (11, 12, 13) to a connecting pin (52) for anchor points, and a curved blade (7) forming, with the inner surface (5) of the hook member (4), a U-shaped profile to surround part of a vertebra, with one traction arm (8) generally opposite and parallel to the inner bearing surface (5) of the hook member (4) to which it is connected by a linking arm (9),
the clip tooth or teeth (22-25) fixedly connected to the clip meter (21) extend, inside the U-shaped profile, towards the traction arm (8) substantially perpendicularly to the inner bearing surface (5) of the hook member (4) to engage in said part of a vertebra in the retaining position.

6. Device according to claim 5, characterised in that the stabilising clip (3) comprises two lateral teeth (22, 23) projecting beyond the side edges (31, 32) of the hook member (4), and at least one frontal tooth (24, 25) projecting beyond the free end edge (33) of the hook member (4).

7. Device according to claim 5, characterised in that the stabilising clip (3) comprises two side teeth (22, 23) projecting beyond the side edges (31, 32) of the hook member (4), and two front teeth (24, 25) projecting beyond the free end edge (33) of the hook member (4).

8. Device according to any one of claims 5 to 7, characterised in that the teeth (22, 25) have a length such that, in the position in which the clip (3) is held on the hook (1), the free ends of the teeth are slightly recessed relative to the traction arm (8) of the hook (1).

9. Device for stabilising an orthopaedic anchor according to any one of claims 1 to 4, wherein:
the anchor is a foot screw (40) comprising an anchor member (41) with an inner bearing surface (5) and an outer fixing surface (6), with an internal threaded rod (70) adapted to screw into the bone of the vertebra, and with means of attachment (11, 12, 13) to a rod (52) for connecting anchor points,
the clip tooth or teeth (22-25), integral with the clip member (21), extend axially around the anchor member (41) and beyond the inner bearing surface (5) to engage in the vertebral bone when the anchor is in position on the vertebra with the clip (3) bearing on the outer fixing surface (6).

10. Apparatus for treating the spine, comprising at least one connecting pin or plate (52) which connects the anchor members adapted to be fixed to the spinal vertebrae, characterised in that at least one of the anchor members comprises an orthopaedic anchor (1) and a device (3) for stabilising an orthopaedic anchor according to any one of claims 1 to 9.

## Patentansprüche

1. Stabilisierungsvorrichtung für eine orthopädische Verankerungsvorrichtung an einem Wirbel für die Bestückung der Wirbelsäule, wobei die Verankerungsvorrichtung folgendes umfaßt:
- einen Verankerungskörper (4, 41) mit einer inneren Anlagefläche (5) zur Anlage an dem Wirbel und einer entgegengesetzten äußeren Befestigungsfläche (6),
- mit dem Verankerungskörper (4, 41) fest verbundene Verankerungsmittel (7, 70), die dafür ausgebildet sind, am Wirbel befestigt zu werden,
- einen mit dem Verankerungskörper (4, 41) fest verbundenen Befestigungsteil (11), der mit Mitteln zur Befestigung an einer Stange oder Platte (52) zur festen Verbindung von Verankerungspunkten versehen ist,
dadurch gekennzeichnet, daß sie eine Stabilisierungsklammer (3) aufweist, die aus folgendem besteht:
einem Klammernkörper (21), der dafür ausgelegt ist, auf die äußere Befestigungsfläche (6) des Verankerungskörpers (4,41) aufgesteckt zu werden, und mit Mitteln (26, 34; 36, 37) zur festen Verbindung mit dem Verankerungskörper (4, 41) versehen ist, die sich den Relativdrehungen zwischen dem Klammernkörper (21) und dem Verankerungskörper (4, 41) widersetzen und wahlweise die Annäherung des Klammernkörpers (21) an den Verankerungskörper (4, 41) bis in eine Stabilisierungsstellung in Anlage an der äußeren Befestigungsfläche (6) und die Entfernung des Klammernkörpers (21) vom Verankerungskörper (4, 41) bis in eine Freigabestellung gestatten,
- mindestens einem Klammerzahn (22-25; 38; 39; 124), der mit dem Klammernkörper (21) fest verbunden ist und so gerichtet und ausgebildet ist, daß er über die innere Anlagefläche (5) des Verankerungskörpers (4, 41) hinaus in den Wirbel eintritt, wenn die Klammer (3) sich in ihrer Stabilisierungsstellung auf dem Verankerungskörper (4, 41) befindet, der seinerseits auf seine Verankerungsstellung auf dem Wirbel eingestellt ist.

2. Vorrichtung nach Anspruch 1, dadurch gekennzeichnet, daß der Klammernkörper (21) an dem Verankerungskörper (4, 41) über eine Querachse (34) angelenkt ist.

3. Vorrichtung nach Anspruch 1, dadurch gekennzeichnet, daß die Stabilisierungsklammer (3) ein von der Verankerungsvorrichtung getrenntes, abnehmbares Teil ist, das dafür ausgelegt ist, auf die äußere Befestigungsfläche (6) des Verankerungskörpers (4, 41) aufgesteckt zu werden.

4. Vorrichtung nach einem der Ansprüche 1 bis 3, dadurch gekennzeichnet, daß
- der Befestigungsteil der Verankerungsvorrichtung eine Gewindestange (11) ist, die an der äußeren Befestigungsfläche (6) des Verankerungskörpers (4, 41) vorsteht und dazu bestimmt ist, einen Klemmreiter (12) und eine Mutter (13) aufzunehmen,
- erste Gleitschutzreliefs (19) auf der äußeren Befestigungsfläche (6) des Verankerungskörpers (4, 41) vorgesehen sind,
- entsprechende Zwischengleitschutzreliefs (29, 30) auf den beiden entgegengesetzten Flächen (27, 28) des Klammernkörpers (21) vorgesehen sind,
- zweite Gleitschutzreliefs (20) auf einer Fläche des Klemmreiters (12) vorgesehen sind.

5. Stabilisierungsvorrichtung einer orthopädischen Verankerungsvorrichtung (1) nach einem der Ansprüche 1 bis 4, in der
- die Verankerungsvorrichtung ein Haken (1) ist, der einen Hakenkörper (4) aufweist, der eine innere Anlagefläche (5) und eine äußere Befestigungsfläche (6) besitzt, die mit Mitteln (11, 12, 13) zur Befestigung an einer Stange (52) zur festen Verbindung von Verankerungspunkten versehen ist, sowie ein gekrümmtes Blatt (7), das mit der inneren Fläche (5) des Hakenkörpers (4) ein U-Profil zur Umhüllung eines Wirbelteils bildet, mit einem Zugschenkel (8), der zur inneren Anlagefläche (5) des Hakenkörpers (4) allgemein entgegengesetzt und parallel ist und mit dieser durch einen Verbindungsschenkel (9) verbunden ist,
- der oder die mit dem Klammernkörper (21) fest verbundenen Klammerzähne (22-25) sich im Inneren des U-Profils in Richtung auf den Zugschenkel (8) im wesentlichen senkrecht zur inneren Anlagefläche (5) des Hakenkörpers (4) erstrecken, um in der Haltestellung in diesen Wirbelteil einzutreten.

6. Vorrichtung nach Anspruch 5, dadurch gekennzeichnet, daß die Stabilisierungsklammer (3) zwei Seitenzähne (22, 23) aufweist, die an den Seitenrändern (31, 32) des Hakenkörpers (4) vorstehen, sowie mindestens einen Frontzahn (24, 25), der am freien Endrand (33) des Hakenkörpers (4) vorsteht.

7. Vorrichtung nach Anspruch 5, dadurch gekennzeichnet, daß die Stabilisierungsklammer (3) zwei Seitenzähne (22, 23) aufweist, die an den Seitenrändern (31, 32) des Hakenkörpers (4) vorstehen, sowie zwei Frontzähne (24, 25), die am freien Endrand (33) des Hakenkörpers (4) vorstehen.

8. Vorrichtung nach einem der Ansprüche 5 bis 7, dadurch gekennzeichnet, daß die Zähne (22, 25) eine solche Länge besitzen, daß ihr freies Ende in der Haltestellung der Klammer (3) auf dem Haken (1) von dem Zugschenkel (8) des Hakens (1) leicht zurückversetzt ist.

9. Stabilisierungsvorrichtung einer orthopädischen Verankerungsvorrichtung nach einem der Ansprüche 1 bis 4, bei der
- die Verankerungsvorrichtung eine Pedikularschraube (40) ist, die einen Verankerungskörper (41) mit einer inneren Anlagefläche (5) und einer äußeren Befestigungsfläche (6) aufweist, sowie eine innere Gewindestange (70), die dafür ausgelegt ist, in den Wirbelknochen eingeschraubt zu werden, und Mitteln (11, 12, 13) zur Befestigung an einer Stange (52) zur festen Verbindung von Verankerungspunkten,
- der oder die mit dem Klammernkörper (21) fest verbundenen Klammerzähne (22-25) sich axial um den Verankerungskörper (41) herum und über die innere Anlagefläche (5) hinaus erstrekken, um in den Wirbelknochen einzutreten, wenn die Verankerungsvorrichtung auf dem Wirbel in Stellung ist, wobei die Klammer (3) auf der äußeren Befestigungsfläche (6) aufliegt.

10. Vorrichtung zur Behandlung der Wirbelsäule, bestehend aus mindestens einer Stange oder Platte (52) zur festen Verbindung, die Verankerungselemente verbindet, die dafür ausgelegt sind, an den Wirbeln der Wirbelsäule befestigt zu werden, dadurch gekennzeichnet, daß mindestens eines der Verankerungselemente eine orthopädische Verankerungsvorrichtung (1) und eine Stabilisierungsvorrichtung (3) einer orthopädischen Verankerungsvorrichtung nach einem de Ansprüche 1 bis 9 umfaßt.
